# EUROPÄISCHE PATENTSCHRIFT

(11) **EP 3 285 790 B1**
(45) Veröffentlichungstag und Bekanntmachung des Hinweises auf die Patenterteilung: **29.07.2020**
(21) Anmeldenummer: 16738074.0
(22) Anmeldetag: 08.06.2016
(51) Int. Cl.: A61K 36/898, A61K 45/06, A61K 9/00, A61K 31/08, A61K 31/155, A61K 31/728, A61K 31/731, A61K 36/04, A61K 36/09, A61K 36/185, A61K 36/48, A61K 36/68, A61K 36/73, A61P 11/04, A61P 11/14

(54) **ZUSAMMENSETZUNG FÜR DIE BEHANDLUNG DES HALS-/RACHENRAUMS**
COMPOSITION FOR TREATING THE THROAT/PHARYNX
COMPOSITION POUR LE TRAITEMENT DE LA CAVITÉ LARYNGOPHARYNGÉE

(30) Priorität: 17.08.2015 DE 102015113521
(43) Veröffentlichungstag der Anmeldung: 28.02.2018
(73) Patentinhaber: Maria Clementine Martin Klosterfrau Vertriebsgesellschaft mbH, 50670 Köln (DE)
(72) Erfinder: GALÁN SOÚSA, José, 50670 Köln (DE); VESTWEBER, Anna-Maria, 51399 Burscheid (DE)
(74) Vertreter: Strehlke, Ingo Kurt
(86) Internationale Anmeldenummer: PCT/EP2016/062949
(87) Internationale Veröffentlichungsnummer: WO 2017/028977

(56) Entgegenhaltungen:
- WO-A1-2016/130830
- WO-A1-2017/028976
- DE-U1-202004 016 646
- DE-U1-202006 008 541
- DE-U1-202008 016 832
- DE-U1-202012 001 752
- DE-U1-202013 000 377
- DE-U1-202013 000 446
- DATABASE GNPD [Online] MINTEL; Oktober 2014 (2014-10), anonymous: "Anginetten", XP002761487, gefunden im www.gnpd.com accession no. 2707871 Database accession no. 2707871
- Anonymous: "Gebrauchsinformation Anginetten Hals-Spray", , 14. Juli 2016 (2016-07-14), Seiten 1-2, XP055300256, Gefunden im Internet: URL:http://www.klosterfrau.de/fileadmin/pr oductdata/anginetten/anginetten_hs/gebrauc hsinformation_anginetten_hals_spray.pdf [gefunden am 2016-09-06]

## Beschreibung

Die vorliegende Erfindung betrifft das medizinische Gebiet der Therapie von entzündlichen Erkrankungen des Mundraums bzw. des Hals- und Rachenraums.

Die Erfindung wird durch die Ansprüche definiert. Alle davon abweichenden Aspekte und Gegenstände werden hier nur für erläuternde Zwecke beschrieben - Feststellungen, dass diese Teil der Erfindung o.ä. seien, müssen im Kontext der ursprünglich eingereichten Anmeldung gesehen werden und ändern den Schutzbereich nicht.

Insbesondere betrifft die vorliegende Erfindung eine Zusammensetzung zur Verwendung bei der Behandlung von entzündlichen Erkrankungen des Mundraums bzw. des Hals- und Rachenraums.

Entzündliche Erkrankungen des Mund- und Rachenraums, d. h. Entzündungen im Bereich der Mundhöhle und des Hals-/Rachenraums, treten oftmals als Begleiterscheinung von Erkältungserkrankungen und grippalen Infekten, aber auch als eigenständige Erkrankungen auf.

Nicht beschränkende Beispiele für derartige entzündliche Erkrankungen des Mundraums sowie des Hals-/Rachenraums sind z. B. Halsentzündungen, insbesondere Angina, Entzündungen des Kehlkopfs (Laryngitis), Entzündungen der Rachenschleimhaut (Pharyngitis) und Entzündungen der Rachenmandeln (Tonsillitis). Auch Entzündungen im Bereich der Mundhöhle, wie z. B. Stomatitis, Gingivitis, Mundschleimhautläsionen oder dergleichen, zählen zu den vorgenannten Erkrankungen des Mund- und Rachenraums.

Für weitergehende diesbezügliche Einzelheiten zu den vorgenannten Erkrankungen kann beispielsweise auf Roche-Lexikon Medizin, 3. Auflage, 1993, Urban & Schwarzenberg, München/Wien/Baltimore, sowie auf Pschyrembel, Medizinisches Wörterbuch, 257. Auflage, 1993, Nikol Verlagsgesellschaft mbH, Hamburg, verwiesen werden.

Der Begriff der entzündlichen Erkrankungen des Mund- und Rachenraums ist somit sehr weit zu verstehen und umfasst sämtliche entzündliche Erkrankungen, welche im Bereich der Mundhöhle, des Rachens und des Halsraums auftreten können.

Die Behandlung entzündlicher Erkrankungen des Mund- und Rachenraums erfolgt - in Abhängigkeit von der Schwere des Krankheitsbildes - im Allgemeinen durch eine topische Therapie und insbesondere in schwerwiegenderen Fällen gegebenenfalls zusätzliche systemische Therapie. So kann in schwereren Fällen eine systemische Verabreichung von Antibiotika vorgenommen werden. In leichteren Fällen kann zur topischen, symptomatischen Behandlung eine alleinige Verabreichung von Antiseptika und entzündungshemmenden Wirkstoffen (z. B. Antiphlogistika, antiinflammatorisch wirkenden Mittel, Lokalantibiotika etc.) vorgesehen sein. Die Wirkstoffe können beispielsweise in Form von Rachenspülungen, Sprays oder Lutschtabletten verabreicht werden.

Als Wirkstoff zur topischen Behandlung von entzündlichen Prozessen bzw. Erkrankungen des Mund- und Rachenraums hat sich insbesondere 1-Hexadecylpyridiniumchlorid (internationaler Freiname: "Cetylpyridiniumchlorid" (CPC)) bewährt. Hierbei handelt es sich um eine quartäre Ammoniumverbindung mit bakterizider und fungizider Wirkung, welche unter anderem in Lutschtabletten zur Anwendung kommt. Nachteilig bei diesem Wirkstoff ist die Tatsache, dass bei hoher Dosierung und übermäßigem Verzehr Magen-Darm-Beschwerden, Atemnot sowie eine vermehrte Methämoglobinbildung, insbesondere bei Kindern, auftreten kann.

Zudem kann als topisches Antiseptikum bzw. Desinfektionsmittel für die Mund-, Hals- und Rachenschleimhaut auch 1,3-Bis(2-ethylhexyl)-hexahydro-5-methyl-5-pyridinamin (internationaler Freiname: "Hexetidin") verabreicht werden, welches beispielsweise als Spray oder in Form von Spül- bzw. Gurgellösungen appliziert werden kann. Bei längerer Anwendung und starker Dosierung kann es auch hierzu Magen-Darm-Beschwerden und darüber hinaus zu Geschmacksirritationen kommen. Zudem geht die Anwendung insbesondere in Form Gurgellösungen teilweise mit dem Problem von Verfärbungen an den Zähnen einher.

Des Weiteren kommen Wirkstoffe natürlichen Ursprungs zum Einsatz, so z. B. sogenannte Schleimdrogen oder deren Extrakte, wie z. B. Isländisches Moos (*Lichen islandicus*). So beschreibt beispielsweise die WO 2006/111258 A1 ein reizlinderndes Hustenmittel, welches eine Kombination von Isländischem Moos, Malve und einer Zinkverbindung enthält. Derartige Präparate bieten aber nicht immer den gewünschten Therapieerfolg.

Weiterhin betrifft die DE 20 2012 001 752 U1 eine pharmazeutische Zusammensetzung in Form einer festen Dosierung, welche zur topischen Behandlung von entzündlichen Erkrankungen des Mund- und Rachenraums eingesetzt wird. Die Zusammensetzung enthält als Wirkstoffe mindestens eine Schleimdroge und im Übrigen Polidocanol als Lokalanästhetikum.

Darüber hinaus gehen die im Stand der Technik bekannten Zusammensetzungen zur Behandlung von entzündlichen Erkrankungen des Hals- und Rachenraumes oftmals mit zwar zeitlich begrenzten, dennoch äußerst unangenehmen Veränderungen des Geschmackssinns bzw. mit Geschmacksirritationen einher.

Vor dem Hintergrund des zuvor geschilderten Standes der Technik liegt der vorliegenden Erfindung die Aufgabe zugrunde, eine Zusammensetzung zur prophylaktischen und/oder therapeutischen Behandlung von entzündlichen Erkrankungen des Mund- und/oder Hals-/Rachenraums bereitzustellen, welche die zuvor geschilderten Probleme des Standes der Technik überwindet oder aber zumindest abschwächt.

Insbesondere liegt der vorliegenden Erfindung die Aufgabe zugrunde, eine Zusammensetzung bereitzustellen, welche sich im Rahmen der Behandlung von entzündlichen Erkrankungen des Mund- und Hals-/Rachenraums durch eine hervorragende Wirkeffizienz sowie durch eine langanhaltende Wirkdauer bei gleichzeitig guter Verträglichkeit auszeichnet.

Zur Lösung des zuvor geschilderten Problems schlägt die vorliegende Erfindung - gemäß einem ersten Aspekt der vorliegenden Erfindung - eine Zusammensetzung, insbesondere eine pharmazeutische Zusammensetzung, gemäß den diesbezüglichen unabhängigen Ansprüchen vor; weitere, insbesondere vorteilhafte Ausgestaltungen der erfindungsgemäßen Zusammensetzung sind Gegenstand der diesbezüglichen Unteransprüche.

Zudem betrifft die vorliegende Erfindung - gemäß einem weiteren Aspekt der vorliegenden Erfindung - eine Applikations- bzw. Verabreichungsvorrichtung gemäß dem diesbezüglich unabhängigen Anspruch.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Bei allen nachstehend genannten relativen bzw. prozentualen gewichtsbezogenen Angaben, insbesondere Mengenangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder einzelfallbedingt - von den nachfolgend angeführten Gewichts-, Mengen- und Bereichsangaben abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung ist somit - gemäß einem **ersten** Aspekt vorliegenden Erfindung - eine Zusammensetzung in Form einer flüssigen Dosierung zur Verwendung bei der prophylaktischen oder therapeutischen Behandlung von entzündlichen Erkrankungen des Mundraums und/oder des Hals-/- Rachenraums, wobei die Zusammensetzung in Kombination und in jeweils wirksamen Mengen
(a) Eibisch (*Althaea officinalis L.*) als Schleimdroge
   in einer Menge im Bereich von 0,01 bis 30 Gew.-%, bezogen auf die Zusammensetzung,
(b) Polidocanol als Lokalanästhetikum
   in einer Menge im Bereich von 0,001 bis 0,5 Gew.-%, bezogen auf die Zusammensetzung,
(c) Polyhexanid als Oberflächendesinfektionsmittel
   in einer Menge im Bereich von 0,001 bis 0,5 Gew.-%, bezogen auf die Zusammensetzung,
enthält,
wobei die Zusammensetzung die Wirk- und/oder Inhaltsstoffe zusammen mit einem pharmakologisch und/oder physiologisch unbedenklichen Träger enthält und wobei der Träger eine Kombination auf Basis von Wasser und Propylenglykol ist und das gewichtsbezogene Verhältnis von Wasser zu Propylenglykol im Bereich von 10 : 1 bis 1 : 10 liegt,
wobei die Schleimdroge in Form eines Flüssigextraktes und/oder Fluidextraktes mit einem Droge/Extrakt-Verhältnis im Bereich von 0,9 : 1 bis 3 : 1 eingesetzt ist, und
wobei die Zusammensetzung ein Konservierungsmittel enthält, wobei das Konservierungsmittel Kaliumsorbat ist.

Im Rahmen der vorliegenden Erfindung ist es auf Basis der erfindungsgemäßen Zusammensetzung mit der ternären Wirkstoffkombination überraschend gelungen, eine schnell eintretende und äußerst langanhaltende Wirkung bei der Behandlung von entzündlichen Erkrankungen des Mund- und Hals-/Rachenraums bereitzustellen. Insbesondere kann mit der erfindungsgemäßen Zusammensetzung die Schmerz- bzw. Entzündungssymptomatik hocheffizient gelindert werden. Darüber hinaus werden Beeinträchtigungen der Phonation und somit der Stimme schnell gelindert und es wird dem unangenehmen Symptom der Mundtrockenheit vorbeugt.

Der Begriff "pharmazeutische Zusammensetzung" bzw. "pharmazeutische Zubereitung", wie er im Rahmen der vorliegenden Erfindung verwendet wird, ist dabei sehr breit zu verstehen und umfasst nicht nur pharmazeutische Präparate bzw. Pharmazeutika und Arzneimittel als solche, sondern auch sogenannte Medizinprodukte, Lebensmittel oder Nahrungsergänzungsmittel.

Bei den im Rahmen der vorliegenden Erfindung eingesetzten Schleimdrogen handelt es sich insbesondere um aus Pflanzen, Pilzen, Tieren oder Mikroorganismen gewonnene Drogen, welche wasserbindende Biopolymere, insbesondere Polysaccharide, als Inhaltsstoffe enthalten. Diese Biopolymere bzw. Polysaccharide werden auch als Schleimstoffe bzw. Schleime bezeichnet.

Schleimstoffe bzw. Schleime sind üblicherweise Heteropolysaccharide mit Molekulargewichten von ca. 50.000 bis 2.000.000, welche durch Extraktion mit heißem oder kaltem Wasser aus der Droge gewonnen werden. Die hochviskosen Lösungen sind im Gegensatz zu den Gummen nicht klebrig. Man unterscheidet saure und neutrale Schleime. Je nach ihrer Zuckerzusammensetzung lassen sie sich in Glucomannane oder Mannane, in Galactomannane, Xylane oder Rhamnogalacturonane einteilen. Nach ihrer Lokalisierung in der Pflanze kann man Vakuolenschleime und Membranschleime unterscheiden. Als Prototyp für einen sauren Pflanzenschleim kann der Schleim der Eibischwurzel angesehen werden. Er enthält L-Rhamnose, D-Galactose, D-Galacturonsäure und D-Glucuronsäure im molaren Verhältnis von ungefähr 3 : 2 : 3 : 3. Der am einfachsten gebaute Teil des Polysaccharids besteht aus sich wiederholenden Undecasaccharid-Untereinheiten.

Erfindungsgemäß ist es vorgesehen, Schleimdrogen auf Basis von Eibisch einzusetzen.

Weiterhin enthält die Zusammensetzung nach der vorliegenden Erfindung mindestens ein Lokalanästhetikum. Als Lokalanästhetika werden im medizinischen Sprachgebrauch im Allgemeinen Anästhetika bezeichnet, welche lediglich eine örtlich begrenzte betäubende Wirkung entfalten. Erfindungsgemäß ist das Lokalanästhetikum Polidocanol.

Bei Polidocanol handelt es sich gemäß der Definition des Europäischen Arzneibuchs 6.0 um ein Gemisch von Ethern verschiedener Macrogole mit Fettalkoholen, hauptsächlich Laurylalkohol. Synonym wird Polidocanol auch als Hydroxypolyethoxydodecan, Macrogollaurylether oder Lauromacrogol 400 bezeichnet. Aufgrund des lipophilen Dodecylrests und der hydrophilen Etherkette lässt sich Polidocanol gut mit Wasser mischen. Darüber hinaus besitzt Polidocanol oberflächenaktive Eigenschaften. Im Stand der Technik wird Polidocanol im Gebiet der Medizin insbesondere zur Verödung von Hämorrhoiden oder Besenreisern in das betroffene Gewebe injiziert; darüber hinaus kann Polidocanol im Stand der Technik in Hautsalben zur Schmerz- und Juckreizlinderung der äußeren Haut (Lederhaut bzw. Dermis) verwendet werden. Im Rahmen der vorliegenden Erfindung kann überraschenderweise erstmals eine topische Applikation von Polidocanol ausgehend von einem Spray auf der empfindlichen Schleimhaut realisiert werden, insbesondere im Zusammenwirken mit den übrigen Komponenten wird in vollkommen überraschender Weise keinerlei Reizung der Schleimhäute beobachtet. Darüber hinaus besitzt Polidocanol in Kombination mit den übrigen erfindungsgemäßen Komponenten eine hervorragende mucoadhäsive Wirksamkeit, was insbesondere zu einer langanhaltenden Wirksamkeit führt. Für weitergehende Einzelheiten zum Wirkstoff "Polidocanol" kann verwiesen werden auf Römpp, Chemielexikon, 10. Auflage, Georg Thieme Verlag, Stuttgart / New York, Band 5, Seite 3403, Stichwort: "Polidocanol", sowie auf die darin referierte Literatur, deren diesbezüglicher Inhalt hiermit vollumfänglich durch Bezugnahme eingeschlossen ist.

Schließlich enthält die erfindungsgemäße Zusammensetzung insbesondere zu Zwecken der Reduzierung der Keimzahl auf der Mund- und Rachenschleimhaut mindestens ein Oberflächendesinfektionsmittel. In diesem Zusammenhang ist es vorgesehen, dass als Oberflächendesinfektionsmittel Polyhexanid eingesetzt wird, wobei es sich hierbei insbesondere um Polyhexamethylenbiguanid (PHNB) handelt.

Bei Polyhexanid handelt es sich um ein gängiges Antiseptikum zur Wundbehandlung. Vorteilhaft an Polyhexanid ist insbesondere sein breites Wirkungsspektrum, welches auch gegen schwer zu behandelnde Krankenhauskeime, wie beispielsweise *Staphylococcus aureus,* gerichtet ist. Darüber hinaus wirkt Polyhexanid bereits in geringen Konzentrationen, da es direkt an die bakterielle Zellwand bindet und diese derart schädigt, dass es zum Zelltod kommen kann. Auf diese Weise wird der Einsatz geringer (Wirk-)Konzentrationen möglich, was auch das Auftreten von Nebenwirkungen minimiert. Darüber hinaus zeichnet sich Polyhexanid durch eine geringe systemische Resorption aus, so dass auch hierdurch Nebenwirkungen weitergehend verringert werden.

Die vorliegende Erfindung weist zahlreiche weitere Vorteile und Besonderheiten auf, welche sie gegenüber dem Stand der Technik auszeichnen und welche nachfolgend ausführlich geschildet sind:
Mit der erfindungsgemäßen Zusammensetzung, welche vorzugsweise als flüssige Dosierung in Form eines Sprays vorliegt, lässt sich in effizienter Weise auch die mit entzündlichen Erkrankungen des Mund- und Hals-/Rachenraums verbundene Schmerzsymptomatik lindern. Bereits nach einmaliger Anwendung bzw. Applikation werden Halsschmerzen, insbesondere wie sie im Rahmen von grippalen Infekten auftreten, deutlich gelindert. Darüber hinaus tritt die schmerzlindernde Wirkung nicht nur sehr schnell ein, sondern hält auch über einen großen Zeitraum an, so dass neben der hohen Wirkeffizienz bzw. Wirksamkeit eine große Wirkdauer vorliegt.

Weiterhin können auf Basis der erfindungsgemäßen ternären Wirkstoffkombination auch Entzündungen im Mund- und Rachenraum, welche oftmals im Zusammenhang mit grippalen Infekten auftreten, gelindert werden.

Die höhere Wirkeffizienz der erfindungsgemäßen Zusammensetzungen resultiert unter anderem - ohne sich hierbei auf diese Theorie beschränken zu wollen - aus den hervorragenden mucoadhäsiven Eigenschaften der Zusammensetzungen. Insbesondere durch den Einsatz mindestens einer Schleimdroge wird eine Art Schutzfilm auf der Mund- und Rachenschleimhaut ausgebildet, so dass einerseits eine physikalische Barriere gegenüber Krankheitserregern, wie Bakterien und Viren, gebildet wird und andererseits einem Austrocknen der Schleimhaut vorgebeugt wird. Darüber hinaus wird auf Basis der guten Anheftung der Zusammensetzung an die Schleimhaut die Wirksamkeit der erfindungsgemäßen Zusammensetzung insgesamt verlängert, da die Wirkstoffe länger am Wirkort verbleiben und somit über einen längeren Zeitraum ihre Wirkung entfalten können. Die mucoadhäsive Wirkung kann zudem durch den zusätzlichen Einsatz von Propylenglykol noch weiterführend verbessert werden.

Durch den erfindungsgemäß vorgesehenen Einsatz mindestens eines Oberflächendesinfektionsmittels auf Basis von Polyhexanid wird in diesem Zusammenhang zudem eine Art Doppelwirkung in Bezug auf einen Schutz gegenüber Krankheitserregern erzielt, insbesondere da der zuvor beschriebene Schutzfilm - ohne sich auf diese Theorie beschränken zu wollen - eine physikalische Barriere für Krankheitserreger darstellt und das Oberflächendesinfektionsmittel zudem eine chemische Barriere für die Krankheitserreger darstellt. Auf Basis der erfindungsgemäßen Zusammensetzung wird somit ein vollumfänglicher Schutz gegenüber grippalen Infekten auslösenden Viren und Bakterien gewährleistet.

Darüber hinaus ermöglicht die erfindungsgemäße Zusammensetzung eine äußerst schnelle Regeneration bzw. Wiederherstellung der gestörten Phonation, d. h. der stimmlichen Beeinträchtigungen. Insbesondere Heiserkeit kann in besonders effizienter Weise behandelt werden.

Die erfindungsgemäße Zusammensetzung zeichnet sich gegenüber bekannten Maßnahmen zur Linderung von Halsschmerzen, Heiserkeit und Entzündungssymptomen im Mund- und Rachenraum durch eine schnell eintretende und lang anhaltende Wirkung aus, d. h. die Wirkeffizienz ist insgesamt verbessert. Darüber hinaus kann besonders effizient einem Austrocken der Schleimhäute vorgebeugt bzw. entgegengewirkt werden.

Insgesamt ergänzen sich somit die erfindungsgemäß eingesetzten Schleimdrogen, das mindestens eine Lokalanästhetikum und das Oberflächendesinfektionsmittel in synergistischer Weise, da die Wirkung der eingesetzten Komponenten bei kombiniertem Einsatz über die Wirkung der jeweiligen Einzelstoffe bei deren alleinigem Einsatz hinausgeht. Insbesondere in Bezug auf eine Barrierewirkung gegenüber Krankheitserregern, eine Linderung der Schmerz- und Entzündungssymptomatik sowie einer Vorbeugung vor Austrocknung der Schleimhäute ergänzen sich die Inhaltsstoffe synergistisch.

Schließlich zeichnet sich die erfindungsgemäße Zusammensetzung auch durch ihre hervorragende Verträglichkeit aus, da sie zumindest im Wesentlichen frei von etwaigen Nebenwirkungen ist. Aufgrund der guten Verträglichkeit ist die erfindungsgemäße Zusammensetzung somit auch für einen länger andauernden Gebrauch, z. B. bei chronischer Mundtrockenheit, wie sie beispielsweise bei Rauchern auftritt, sowie grundsätzlich auch zur Anwendung bei Kindern geeignet.

Im Zusammenhang mit den zuvor beschriebenen Vorteilen und Besonderheiten der vorliegenden Erfindung wird bereits an dieser Stelle auf die von der Anmelderin durchgeführten und nachfolgend geschilderten Wirksamkeitsstudien verwiesen, welche die vorgenannten Effekte und Eigenschaften in eindrucksvoller Weise belegen.

Die erfindungsgemäße Zusammensetzung kann in vielfältiger Art und Weise ausgestaltet sein, wobei bevorzugte Ausführungsformen im Folgenden näher beschrieben sind.

Im Rahmen der vorliegenden Erfindung ist es üblicherweise vorgesehen, dass die Zusammensetzung die Wirk- und/oder Inhaltsstoffe zusammen mit einem unbedenklichen, insbesondere pharmakologisch und/oder physiologisch unbedenklichen Träger und/oder Exzipienten enthält.

Zudem ist es im Rahmen der vorliegenden Erfindung vorgesehen, dass der Träger eine Kombination von Wasser und Propylenglykol ist.

Durch den gegebenenfalls vorgesehenen Einsatz eines Trägers bzw. Exzipienten lässt sich die erfindungsgemäße Zusammensetzung weiterführend insbesondere in Bezug auf die Haftungseigenschaften der Wirkstoffe an der Mund- bzw. Rachenschleimhaut modifizieren, was wiederum auch die Wirkdauer der Zusammensetzung verlängert. Eine besonders gute mucoadhäsive Wirkung, d. h. eine gute Anheftung der Zusammensetzung und der darin enthaltenen Wirkstoffe an die Schleimhaut, wird mit dem erfindungsgemäßen Träger auf Basis einer Kombination von Wasser und Propylenglykol erzielt. Darüber hinaus trägt Propylenglykol zu einer verbesserten Konservierung der Zusammensetzung bei. In diesem Zusammenhang ist es zudem vorgesehen, dass das gewichtsbezogene Verhältnis von Wasser zu Propylenglykol im Bereich von 10 : 1 bis 1 : 10 liegt. Der Wasseranteil gewährleistet eine komfortable Sprüh- bzw. Applizierbarkeit der erfindungsgemäßen Zusammensetzungen, wohingegen der Anteil an Propylenglykol die Haftung der Zusammensetzung an der Schleimhaut sowie die Konservierung unterstützt.

Was die eingesetzte Menge des Trägers bzw. Exzipienten anbelangt, so kann diese im Allgemeinen in weiten Bereichen variieren: Üblicherweise enthält die Zusammensetzung gemäß der vorliegenden Erfindung den Träger in einer Menge im Bereich von 0,1 bis 90 Gew.-%, insbesondere im Bereich von 0,5 bis 50 Gew.-%, vorzugsweise im Bereich von 1 bis 30 Gew.-%, bevorzugt im Bereich von 5 bis 20 Gew.-%, besonders bevorzugt im Bereich von 7 bis 15 Gew.-%, bezogen auf die Zusammensetzung.

In Bezug auf die Wirkung bzw. Wirkeffizienz der erfindungsgemäßen Zusammensetzung ist es zudem vorgesehen, dass die Schleimdroge in Form eines Flüssigextraktes und/oder Fluidextraktes eingesetzt ist. In diesem Zusammenhang ist es im Rahmen der vorliegenden Erfindung bevorzugt, wenn die Schleimdroge in Form eines wässrigen, alkoholischen oder wässrig-alkoholischen Extrakts eingesetzt ist.

Im Rahmen der vorliegenden Erfindung ist es vorgesehen, dass die Schleimdroge in Form eines Flüssigextraktes mit einem Droge/Extrakt-Verhältnis im Bereich von 0,9 : 1 bis 3 : 1, noch mehr bevorzugt von etwa 1 : 1, eingesetzt ist.

Das sogenannte Droge/Extrakt-Verhältnis (DEV) charakterisiert das (gewichtsbezogene) Verhältnis von eingesetzter Ausgangsdroge zu erhaltenem Extrakt. Das Droge/Extrakt-Verhältnis (DEV) gibt also an, aus welcher gewichtsbezogenen Menge an eingesetzter Droge (z. B. Eibischwurzel) welche gewichtsbezogene Menge an Extrakt gewonnen ist. Ein Droge/Extrakt-Verhältnis von beispielsweise 2 : 1 bedeutet, dass aus zwei Gewichtsteilen Droge ein Gewichtsteil Extrakt gewonnen wurde. Das Droge/Extrakt-Verhältnis gibt also an, wie viele Gewichtsteile einer Arzneidroge für die Herstellung des gewichtsbezogenen Extraktäquivalents benötigt werden.

Die Verwendung von Extrakten mit definiertem Droge/Extrakt-Verhältnis ist insbesondere vor dem Hintergrund relevant, dass die Qualität des Extraktes einen hohen Einfluss auf die Gesamtqualität der pharmazeutischen Zusammensetzung hat. Vorteilhaft ist es dabei zudem, dass der Extrakt hinsichtlich der Wirkstoffkonzentration gewissermaßen standardisiert ist, so dass auch die finale pharmazeutische Zubereitung qualitativ und quantitativ mit konstant guten bzw. gleichbleibenden Werten bezüglich der Wirk- und Inhaltsstoffe bereitgestellt werden kann.

Was darüber hinaus die im Rahmen der vorliegenden Erfindung eingesetzte Schleimdroge im Speziellen anbelangt, so ist diese erfindungsgemäß Eibisch (*Althaea officinalis L.).*

Der Eibisch (*Althaea officinalis L.*), welcher vorzugsweise in Form der Eibischwurzel, -blätter und -blüten eingesetzt wird, wächst auf salzhaltigen und feuchten Böden Mittel-, Ost- und Südosteuropas. Die im Herbst geernteten, von der holzigen Hauptwurzel, von Wurzelfasern und Rindenschichten befreiten und bei 35 °C getrockneten Wurzelzweige und Nebenwurzeln kommen geschält oder ungeschält in den Handel. Die Braunfärbung der geschälten Droge bedeutet eine Qualitätsminderung; ein nachträgliches "Schönen" mit Sulfitlösung ist unzulässig. Weiterhin kommen die vor oder während der Blüte gesammelten, etwa 10 cm langen, etwa 8 cm breiten, drei- bis fünflappigen, graufilzig behaarten getrockneten Blätter und die im Juli/August gesammelten, fleischfarbenen getrockneten Kronblätter zur Anwendung. Der Eibisch ist eine mehrjährige, etwa 1 m hohe Staude, die generativ oder vegetativ vermehrt wird. Die im Spätherbst geerntete Wurzel enthält bis zu 15 % Schleimstoffe. Im Frühjahr und Sommer liegt der Schleimgehalt bei 5 bis 6 %. Der Schleimgehalt der Blatt- und Blütendroge beträgt 6 bis 9 %. Der Schleim ist in der Wurzel in bestimmten Schleimzellen des Rinden- und Holzparenchyms lokalisiert. Er besteht aus Galacturonorhamnanen, Glucanen und Arabinogalactanen. Wässrige Auszüge aus der Wurzel sollen durch Mazeration bei Zimmertemperatur hergestellt werden, damit die reichlich vorhandene Stärke nicht durch Quellung störend wirkt. Eibischdrogen werden für Teezubereitungen oder zur Herstellung von Sirups Althaeae verwendet. Das Hauptanwendungsgebiet sind entzündliche Reizzustände des Rachenraums. Äußerlich kommt der Eibisch zu erweichenden Umschlägen, Bädern und Kataplasmen zur Anwendung.

Was die eingesetzte Menge an Eibisch anbelangt, so kann diese in weiten Bereichen variieren. Erfindungsgemäß enthält die Zusammensetzung die Schleimdroge in Form von deren Fluidextrakt in einer Menge im Bereich von 0,01 bis 30 Gew.-%, insbesondere im Bereich von 0,1 bis 20 Gew.-%, bevorzugt im Bereich von 1 bis 15 Gew.-%, besonders bevorzugt im Bereich von 8 bis 12,5 Gew.-%, bezogen auf die Zusammensetzung.

Darüber hinaus hat sich gezeigt, dass sich durch den zusätzlichen Einsatz eines Oberflächendesinfektionsmittels die Keimzahl auf der Mund- und Rachenschleimhaut signifikant reduzieren lässt. Die Zusammensetzung enthält als Oberflächendesinfektionsmittel Polyhexanid bzw. dessen pharmazeutisch verträgliche Salze, insbesondere Polyhexanidhydrochlorid.

Was die eingesetzten Mengen des Oberflächendesinfektionsmittels anbelangt, so liegen diese erfindungsgemäß im Bereich von 0,001 bis 5 Gew.-%, insbesondere im Bereich von 0,01 bis 2,5 Gew.-%, vorzugsweise im Bereich von 0,05 bis 2 Gew.-%, bevorzugt im Bereich von 0,01 bis 1 Gew.-%, besonders bevorzugt im Bereich von 0,001 bis 0,5 Gew.-%, bezogen auf die Zusammensetzung.

Darüber hinaus ist es für die Wirksamkeit der erfindungsgemäßen Zusammensetzung vorteilhaft, wenn das eingesetzte Lokalanästhetikum Polidocanol ist.

Zur Erzielung einer effizienten Schmerzlinderung ist es im Rahmen der vorliegenden Erfindung vorgesehen, dass die Zusammensetzung das Lokalanästhetikum in einer Menge im Bereich von 0,001 bis 5 Gew.-%, insbesondere im Bereich von 0,01 bis 2,5 Gew.-%, vorzugsweise im Bereich von 0,05 bis 1 Gew.-%, bevorzugt im Bereich von 0,1 bis 0,5 Gew.-%, bezogen auf die Zusammensetzung, enthält.

Weiterhin kann es im Rahmen der vorliegenden Erfindung vorgesehen sein, dass die Zusammensetzung als weitere Komponente Menthol enthält. Bei Menthol handelt es sich um einen monozyklischen Monotherpen-Alkohol, welcher in zahlreichen ätherischen Ölen, insbesondere in Minzöl aus der Pfefferminze (*Mentha* x *piperita*), vorkommt. Menthol ist aufgrund seines frischen Geschmacks einerseits ein Aromastoff, welcher der Zusammensetzung einen angenehmen frischen Geschmack verleiht. Andererseits besitzt Menthol auf der Schleimhaut eine kühlende, juckreizlindernde, schmerzstillende sowie leicht lokalanästhetische und antimikrobielle Wirkung. Durch den Einsatz von Menthol kann die Wirksamkeit der erfindungsgemäßen Zusammensetzungen noch weiter verbessert werden.

In diesem Zusammenhang kann es vorgesehen sein, dass die Zusammensetzung Menthol in einer Menge im Bereich von 0,001 bis 5 Gew.-%, insbesondere im Bereich von 0,01 bis 2 Gew.-%, bevorzugt im Bereich von 0,02 bis 1 Gew.-%, besonders bevorzugt im Bereich von 0,05 bis 0,1 Gew.-%, bezogen auf die Zusammensetzung, enthält.

Darüber hinaus hat sich im Rahmen der vorliegenden Erfindung gezeigt, dass die Stabilität der Zusammensetzung, insbesondere die Stabilität der Inhaltsstoffe, noch weiterführend gesteigert werden kann, wenn die Zusammensetzung mindestens ein Konservierungsmittel enthält. Darüber hinaus kann die Zusammensetzung durch den Einsatz mindestens eines Konservierungsmittels vor mikrobiellem Befall geschützt werden.

Erfindungsgemäß enthält die Zusammensetzung als Konservierungsmittel Kaliumsorbat. Durch den Einsatz von Kaliumsorbat lassen sich die übrigen Inhaltsstoffe besonders gut stabilisieren. Einerseits bleiben die erfindungsgemäßen Zusammensetzungen bei Einsatz von Kaliumsorbat als Konservierungsmittel besonders lange frei von mikrobiologischem Befall und andererseits ist die Lagerstabilität insgesamt erhöht, d. h. die Zusammensetzungen bleiben auch über einen langen Zeitraum frei von Eintrübungen bzw. Ausflockungen der Inhaltsstoffe und sind somit länger verwendbar.

Was die eingesetzte Menge des Konservierungsmittels anbelangt, so kann diese in weiten Bereichen variieren. Besonders gute Ergebnisse werden im Rahmen der vorliegenden Erfindung erzielt, wenn die Zusammensetzung das Konservierungsmittel in einer Menge im Bereich von 0,001 bis 10 Gew.-%, insbesondere im Bereich von 0,005 bis 5 Gew.-%, vorzugsweise im Bereich 0,01 bis 1 Gew.-%, bevorzugt im Bereich von 0,2 bis 0,3 Gew.-%, bezogen auf die Zusammensetzung, enthält.

Zudem kann es vorgesehen sein, dass die Zusammensetzung mindestens einen weiteren Wirk-, Hilfs-, Additiv- und/oder Inhaltsstoff, insbesondere ausgewählt aus der Gruppe von Verarbeitungshilfsmitteln, Aromastoffen, Geschmacksstoffen, Süßstoffen und Süßungsmitteln, Säuerungsmitteln, Stabilisatoren, Vitaminen, Mineralien und/oder Spurenelementen, enthält.

In Bezug auf die Dosierung der erfindungsgemäßen Zusammensetzung ist es zudem erfindungsgemäß gemäß einer besonders bevorzugten Ausführungsform vorgesehen, dass die Zusammensetzung in Form einer flüssigen Dosierung, vorzugsweise für die insbesondere topische Sprühapplikation, vorliegt. In diesem Zusammenhang ist es bevorzugt, wenn die Zusammensetzung in einem Applikator bzw. einer Applikationsvorrichtung insbesondere für die Sprühapplikation vorliegt. Auf dieser Basis ist eine komfortable und sichere Anwendung der Zusammensetzung möglich. Darüber hinaus ist die Zusammensetzung auch nach dem ersten Öffnen geschützt vor Keimen, was auch vorteilhaft für deren Haltbarkeit ist.

Die zuvor beschriebene Zusammensetzung nach der Erfindung ist somit insbesondere zur Verwendung bei der prophylaktischen und/oder kurativen Behandlung von Heiserkeit und/oder entzündlichen Erkrankungen des Mundraums und/oder des Hals-/ Rachenraums, insbesondere zur Verwendung bei der topischen Behandlung von Heiserkeit oder Halsschmerzen, geeignet.

Wie zuvor beschrieben wurde, zeichnet sich die erfindungsgemäße Zusammensetzung gegenüber den aus dem Stand der Technik bekannten Maßnahmen durch ihren schnellen Wirkeintritt sowie die lang anhaltende Wirkung im Rahmen der Verwendung zur Behandlung von Heiserkeit und/oder entzündlichen Erkrankungen des Mund- und Rachenraums aus. Insbesondere ermöglicht die erfindungsgemäße Verwendung der zuvor beschriebenen Zusammensetzungen eine rasche Linderung der Schmerz- und Entzündungsymptomatik und gewährleistet gleichzeitig einen effizienten Schutz gegenüber Krankheitserregern, wie Bakterien und Viren.

Weiterer Gegenstand der vorliegenden Erfindung - gemäß einem **weiteren** Aspekt der vorliegenden Erfindung - ist schließlich eine Applikations- und/oder Verabreichungsvorrichtung, insbesondere in Form eines Sprühapplikators, wobei die Applikations- und/oder Verabreichungsvorrichtung eine Zusammensetzung, wie sie zuvor definiert wurde, umfasst.

Für weitergehende Einzelheiten zu der erfindungsgemäßen Verwendung kann - zur Vermeidung unnötiger Wiederholungen - auf obige Ausführungen zu der erfindungsgemäßen Zusammensetzung verwiesen werden, welche in Bezug auf die erfindungsgemäße Verwendung entsprechend gelten.

Weitere Ausgestaltungen, Abwandlungen und Variationen sowie Vorteile der vorliegenden Erfindung sind für den Fachmann beim Lesen der Beschreibung ohne Weiteres erkennbar und realisierbar, ohne dass er dabei den Rahmen der vorliegenden Erfindung verlässt.

Die vorliegende Erfindung wird anhand der folgenden Ausführungsbeispiele veranschaulicht, welche die vorliegende Erfindung jedoch keinesfalls beschränken.

### Ausführungsbeispiele:

### A) Herstellung erfindungsgemäßer Zusammensetzung in Form eines Halssprays und von Vergleichszusammensetzungen

Zu Vergleichszwecken werden erfindungsgemäße und nicht erfindungsgemäße Zusammensetzungen in Form von Halsspray hergestellt. Als Träger bzw. Exzipient wird Wasser eingesetzt, wobei die übrigen Inhaltsstoffe in dem Träger gelöst vorliegen. Die Herstellung der sprayförmigen Zusammensetzungen erfolgt nach dem Fachmann bekannten Methoden.

Die erfindungsgemäßen Zusammensetzungen A weisen als pharmakologisch relevante Inhaltsstoffe Eibisch-Fluidextrakt mit einem Drogeextraktverhältnis von 1 : 1, Polidocanol als Lokalanästhetikum sowie Polyhexanid-Hydrochlorid als Oberflächendesinfektionsmittel auf. Der Träger besteht aus einer Kombination von Wasser und Propylenglykol. Die genaue Zusammensetzung ist der nachfolgenden Tabelle 1 zu entnehmen.

**Tabelle 1: Erfindungsgemäße Zusammensetzung A**

| Inhaltsstoff | Menge (Gew.-%) |
|---|---|
| Eibisch-Fluidextrakt (DEV: 1 : 1) | 0,13 |
| Polidocanol | 0,2 |
| Polyhexanid-Hydrochlorid | 0,02 |
| Kaliumsorbat | 0,24 |
| Propylenglykol | 10,00 |
| Wasser | ad 100 |

Darüber hinaus werden erfindungsgemäße Zusammensetzungen B bereitgestellt, welche im Gegensatz zu der erfindungsgemäßen Zusammensetzung A jedoch kein Propylenglykol enthalten. Die Rezeptur von Zusammensetzung B ist der nachstehend aufgeführten Tabelle 2 zu entnehmen.

**Tabelle 2: Erfindungsgemäße Zusammensetzung B**

| Inhaltsstoff | Menge (Gew.-%) |
|---|---|
| Eibisch-Fluidextrakt (DEV: 1 : 1) | 0,13 |
| Polidocanol | 0,2 |
| Polyhexanid-Hydrochlorid | 0,02 |
| Kaliumsorbat | 0,24 |
| Wasser | ad 100 |

Weiterhin werden die Vergleichszusammensetzungen C bis H bereitgestellt, welche ebenfalls auf der Rezeptur von Zusammensetzung A basieren. Die Unterschiede zu Zusammensetzung A sind nachfolgend aufgeführt:
Die Vergleichszusammensetzungen C bis E unterscheiden sich in Bezug auf das Lokalanästhetikum von Zusammensetzung A. Die Vergleichszusammensetzung C enthält - anstelle von Polidocanol - Benzocain als Lokalanästhetikum. Die Vergleichszusammensetzung D enthält als Lokalanästhetikum Lidocain. Die Vergleichszusammensetzung E enthält kein Lokalanästhetikum.

Weiterhin werden die Vergleichszusammensetzungen F bis H bereitgestellt, welche sich in Bezug auf das Oberflächendesinfektionsmittel von Zusammensetzung A unterscheiden. Zusammensetzung F enthält anstelle von Polyhexanid Octenidin als Antiseptikum; Vergleichszusammensetzung G enthält Chlorhexidin anstelle von Polyhexanid. Vergleichszusammensetzung H enthält kein Oberflächendesinfektionsmittel.

Weiterhin wird eine Vergleichszusammensetzung I bereitgestellt, welche ebenfalls auf der Rezeptur von Zusammensetzung A basiert, jedoch keine Schleimdroge enthält.

Die Zusammensetzungen J und K unterscheiden sich bezüglich des Konservierungsmittels von Zusammensetzung A. Die Zusammensetzung J enthält kein Konservierungsmittel. Bei Zusammensetzung K wird das in Zusammensetzung A enthaltene Kaliumsorbat durch Benzalkoniumchlorid als Konservierungsmittel ersetzt.

### B) Wirksamkeits- und Anwendungsstudien mit den Halssprays A bis I

Um die Wirksamkeit des erfindungsgemäßen Halssprays zu untersuchen, werden die erfindungsgemäßen Zusammensetzungen A und B mit den Vergleichszusammensetzungen C bis I bezüglich ihrer Wirkeffizienz miteinander verglichen, indem sie im Rahmen von Anwendungs- und Wirksamkeitsstudien verabreicht werden. Darüber hinaus werden zu Vergleichszwecken im Rahmen der Anwendungs- und Wirksamkeitsstudien Gurgellösungen auf Basis von Salbei und Salz eingesetzt.

Zur Untersuchung der Wirksamkeit der Zusammensetzungen A bis I sowie der Gurgellösungen wurde eine Probandengruppe mit insgesamt 100 Probanden im Alter von 20 bis 70 Jahren, von denen 45 weiblich und 55 männlich waren, herangezogen. Die Probanden litten an Heiserkeit sowie entzündlichen Erkrankungen des Hals- und Rachenraumes, welche aus grippalen Infekten resultierten.

Jeweils 10 Probanden erhielten 3 mal täglich über einen Zeitraum von 3 Tagen jeweils eine der Zusammensetzungen A bis I. 10 weitere Probanden erhielten darüber hinaus über einen Zeitraum von ebenfalls 3 Tagen 3 mal täglich eine Gurgellösung auf Basis von Salzwasser und Salbei. Im Rahmen der Behandlung wurde die jeweils getestete Zusammensetzung bzw. Therapiemaßnahme in Bezug auf eine Linderung der Schmerz- und Entzündungssymptomatik, eine Linderung des Symptoms der Mundtrockenheit, die Nachhaltigkeit der Wirkung sowie eine Verbesserung der Phonation nach dem Schulnotensystem (1 = sehr gut bis 6 = ungenügend) bewertet. Die diesbezüglich erhaltenen Ergebnisse sind der nachstehenden Tabelle 3 zu entnehmen.

**Tabelle 3: Ergebnisse der Anwendungs- und Wirksamkeitsstudien**

| **Zusammensetzung** | **Linderung Schmerz symptomatik** | **Linderung Entzündungssymptomatik** | **Linderung Mundtrockenheit** | **Nachhaltigkeit der Wirkung** | **Verbesserung der Phonation** |
|---|---|---|---|---|---|
| A | 1,2 | 1,1 | 1,2 | 1,4 | 1,2 |
| B | 1,4 | 1,6 | 1,5 | 1,7 | 1,4 |
| C | 2,3 | 2,5 | 2,3 | 3,1 | 2,9 |
| D | 2,8 | 2,3 | 2,5 | 2,8 | 3,1 |
| E | 3,9 | 4,2 | 3,4 | 4,1 | 4,2 |
| F | 2,5 | 2,4 | 2,9 | 2,8 | 2,7 |
| G | 2,3 | 2,7 | 2,6 | 3,0 | 2,9 |
| H | 3,7 | 3,9 | 4,0 | 3,7 | 4,1 |
| I | 3,3 | 3,2 | 4,3 | 3,9 | 4,0 |
| Gurgellösung | 4,5 | 4,1 | 4,0 | 4,1 | 4,3 |

Mit den beiden erfindungsgemäßen Zusammensetzungen A und B werden in Bezug auf sämtliche untersuchte Bewertungskriterien hervorragende Ergebnisse erzielt. Die besten Ergebnisse werden insgesamt mit Zusammensetzung A erzielt, welche zusätzlich Propylenglykol enthält. Auf Basis der erfindungsgemäßen Kombination können somit äußerst effizient Hals- und Rachenschmerzen gelindert werden; darüber hinaus kommt es auch in Bezug auf Entzündungssymptome zu einer schnellen Verbesserung. Weiterhin lassen sich mit den erfindungsgemäßen Zusammensetzungen zudem Beeinträchtigungen der Phonation hervorragend behandeln, und auch dem Symptom der Mundtrockenheit kann effektiv vorgebeugt bzw. entgegengestellt werden.

Insgesamt zeichnen sich die erfindungsgemäßen Zusammensetzungen in Bezug auf ihre Wirkung durch eine hervorragende Nachhaltigkeit und Wirkeffizienz aus. Durch den zusätzlichen Einsatz von Propylenglykol kann die Wirksamkeit der erfindungsgemäßen Zusammensetzungen noch weiterführend gesteigert werden, was - ohne sich hierbei auf diese Theorie beschränken zu wollen - vermutlich aus einer Förderung der mucoadhäsiven Wirkung der Zusammensetzung resultiert. Durch Propylenglykol erfolgt eine bessere Haftung der eingesetzten Inhaltsstoffe an der Mund- und Rachenschleimhaut, so dass diese über einen längeren Zeitraum ihre Wirkung am Wirkort entfalten können. Darüber hinaus wird der Schutz gegenüber Krankheitserregern weiterführend verbessert.

Mit den Vergleichszusammensetzungen C bis I sowie der aus dem Stand der Technik bekannten Anwendung von Gurgellösungen können hingegen keine zufriedenstellenden Ergebnisse erzielt werden. Wie aus den Vergleichsbeispielen C und D hervorgeht, wird mit den Lokalanästhetika Benzocain und Lidocain nicht die gleiche hervorragende Wirkung wie mit dem erfindungsgemäß eingesetzten Polidocanol erzielt.

Weiterhin zeigen auch die aus dem Stand der Technik bekannten Antiseptika Octenidin und Chlorhexidin in der erfindungsgemäßen Wirkstoffkombination nicht die gleiche hervorragende Wirksamkeit wie das erfindungsgemäß eingesetzte Polyhexanid, wie aus den mit den Vergleichszusammensetzungen F und G erhaltenen Ergebnissen hervorgeht.

Auch wurden mit den Vergleichszusammensetzungen I sowie mit den Gurgellösungen keine zufriedenstellenden Ergebnisse in Bezug auf die Behandlung von entzündlichen Erkrankungen des Mund- und Rachenraumes erzielt.

Aus den Untersuchungen geht insgesamt hervor, dass die Anwendung der erfindungsgemäßen Zusammensetzungen bei Heiserkeit bzw. entzündlichen Erkrankungen des Mundraums bzw. des Hals- und Rachenraums zu einer signifikanten Linderung der Beschwerden führt; insbesondere tritt eine Linderung der Schmerz- und Entzündungssymptomatik ein. Auch führt die Applikation der erfindungsgemäßen Zusammensetzungen zu einem beschleunigten Rückgang der Beeinträchtigung der Phonation.

### C) Stabilitäts- und Haltbarkeitsuntersuchungen

Weiterhin werden die erfindungsgemäßen Zusammensetzungen A und B, welche Kaliumsorbat und Propylenglykol bzw. Kaliumsorbat enthalten, in Bezug auf ihre Stabilität und Haltbarkeit mit der Zusammensetzung J, welche keinerlei Konservierungsmittel enthält, sowie der Zusammensetzung H, welche Benzalkoniumchlorid als Konservierungsmittel enthält, untersucht. Dazu werden die Zusammensetzungen über einen Zeitraum von 18 Monaten abgedunkelt bei Raumtemperatur, d.h. 22 °C, gelagert. Nach Ablauf von 18 Monaten werden die Zusammensetzungen auf mikrobiellen Befall sowie die Lagerstabilität hin untersucht. Als Indikator für eine verringerte Lagerstabilität wird dabei das Auftreten von Eintrübungen durch ausgefallene Inhaltsstoffe herangezogen. Die diesbezüglich erhaltenen Ergebnisse sind der nachstehenden Tabelle 4 zu entnehmen.

**Tabelle 4: Ergebnisse der Stabilitätsuntersuchungen**

| **Zusammensetzung** | **mikrobieller Befall** | **Eintrübungen** |
|---|---|---|
| A | - | - |
| B | - | + |
| I | ++ | ++ |
| H | - | ++ |

| | | |
|---|---|---|
| -: kein Befall / keine Trübung +: leichter Befall / leichte Trübung, Verwendung noch möglich ++: starker Befall / starke Trübung, Verwendung nicht mehr möglich | | |

Wie die vorstehenden Ausführungen zeigen, werden in Bezug auf die Stabilität und den mikrobiellen Befall die besten Ergebnisse mit den erfindungsgemäßen Zusammensetzungen A erzielt, welche sowohl Kaliumsorbat als Konservierungsmittel als auch Propylenglykol enthalten. Nach 18 Monaten Lagerzeit wird weder ein mikrobieller Befall noch eine Eintrübung der Zusammensetzungen beobachtet. Auch die erfindungsgemäße Zusammensetzung B, welche kein Propylenglykol enthält, ist nach einer Lagerzeit von 18 Monaten noch verwendbar, allerdings treten bereits leichte Eintrübungen auf, was auf eine geringfügig verringerte Stabilität der Inhaltsstoffe schließen lässt.

Die Zusammensetzung J hingegen weist keine zufriedenstellende Stabilität bzw. Haltbarkeit auf. Bereits nach 18 Monaten ist ein ausgeprägter mikrobieller Befall nachweisbar. Darüber hinaus zeigt die Zusammensetzung starke Eintrübungen. Eine Verwendung der Zusammensetzung ist nach 18 Monaten nicht mehr möglich. Zusammensetzung H, welche Benzalkoniumchlorid als Konservierungsmittel enthält, bleibt zwar frei von mikrobiellem Befall, allerdings weist die Zusammensetzung starke Eintrübungen auf, so dass diese ebenfalls nicht mehr verwendbar ist.

Die obigen Ausführungen zeigen, dass sich durch den Einsatz von Kaliumsorbat einerseits und von Propylenglykol andererseits die Stabilität der erfindungsgemäßen Zusammensetzungen signifikant verbessern lässt. Die Zusammensetzungen können in effizienter Weise vor mikrobiellem Befall geschützt werden. Darüber hinaus wird auch die Lagerstabilität bzw. die Stabilität der Inhaltsstoffe in der Lösung verbessert, so dass die Haltbarkeit insgesamt verlängert wird.

## Patentansprüche

1. Zusammensetzung in Form einer flüssigen Dosierung zur Verwendung bei der prophylaktischen oder therapeutischen Behandlung von entzündlichen Erkrankungen des Mundraums und/oder des Hals-/Rachenraums, wobei die Zusammensetzung in Kombination und in jeweils wirksamen Mengen
(a) Eibisch (*Althaea officinalis L.*) als Schleimdroge
in einer Menge im Bereich von 0,01 bis 30 Gew.-%, bezogen auf die Zusammensetzung,
(b) Polidocanol als Lokalanästhetikum
in einer Menge im Bereich von 0,001 bis 0,5 Gew.-%, bezogen auf die Zusammensetzung,
(c) Polyhexanid als Oberflächendesinfektionsmittel
in einer Menge im Bereich von 0,001 bis 0,5 Gew.-%, bezogen auf die Zusammensetzung,
enthält,
wobei die Zusammensetzung die Wirk- und/oder Inhaltsstoffe zusammen mit einem pharmakologisch und/oder physiologisch unbedenklichen Träger enthält und wobei der Träger eine Kombination auf Basis von Wasser und Propylenglykol ist und das gewichtsbezogene Verhältnis von Wasser zu Propylenglykol im Bereich von 10 : 1 bis 1 : 10 liegt,
wobei die Schleimdroge in Form eines Flüssigextraktes und/oder Fluidextraktes mit einem Droge/Extrakt-Verhältnis im Bereich von 0,9 : 1 bis 3 : 1 eingesetzt ist, und
wobei die Zusammensetzung ein Konservierungsmittel enthält, wobei das Konservierungsmittel Kaliumsorbat ist.

2. Zusammensetzung zur Verwendung nach Anspruch 1, wobei die Schleimdroge in Form eines wässrigen, alkoholischen oder wässrig-alkoholischen Extrakts eingesetzt ist.

3. Zusammensetzung zur Verwendung nach Anspruch 1 oder 2, wobei die Schleimdroge in Form eines Flüssigextraktes mit einem Droge/Extrakt-Verhältnis von etwa 1 : 1 eingesetzt ist.

4. Zusammensetzung zur Verwendung nach einem der vorangehenden Ansprüche, wobei die Zusammensetzung die Schleimdroge in Form von deren Extrakt in einer Menge im Bereich von 0,1 bis 20 Gew.-%, bezogen auf die Zusammensetzung, enthält.

5. Zusammensetzung zur Verwendung nach einem der vorangehenden Ansprüche, wobei das Oberflächendesinfektionsmittel Polyhexanidhydrochlorid ist.

6. Zusammensetzung zur Verwendung nach einem der vorangehenden Ansprüche, wobei die Zusammensetzung das Konservierungsmittel in einer Menge im Bereich von 0,001 bis 10 Gew.-%, insbesondere im Bereich von 0,005 bis 5 Gew.-%, vorzugsweise im Bereich 0,01 bis 1 Gew.-%, bevorzugt im Bereich von 0,2 bis 0,3 Gew.-%, bezogen auf die Zusammensetzung, enthält.

7. Zusammensetzung zur Verwendung nach einem der vorangehenden Ansprüche, wobei die Zusammensetzung mindestens einen weiteren Wirk-, Hilfs-, Additiv- und/oder Inhaltsstoff enthält.

8. Zusammensetzung zur Verwendung nach Anspruch 7, wobei der mindestens eine weitere Wirk-, Hilfs-, Additiv- und/oder Inhaltsstoff ausgewählt ist aus der Gruppe von Verarbeitungshilfsmitteln, Aromastoffen, Geschmacksstoffen, Süßstoffen und Süßungsmitteln, Säuerungsmitteln, Stabilisatoren, Vitaminen, Mineralien und/oder Spurenelementen.

9. Zusammensetzung zur Verwendung nach einem der vorangehenden Ansprüche bei der prophylaktischen und/oder kurativen Behandlung von Heiserkeit und/oder entzündlichen Erkrankungen des Mundraums und/oder des Hals-/Rachenraums, insbesondere zur Verwendung bei der topischen Behandlung von Heiserkeit oder Halsschmerzen.

10. Applikations- und/oder Verabreichungsvorrichtung, insbesondere in Form eines Sprühapplikators, wobei die Applikations- und/oder Verabreichungsvorrichtung eine Zusammensetzung, wie in einem der vorangehenden Ansprüche definiert, umfasst.

## Claims

1. A composition in the form of a liquid dose for use in the prophylactic or therapeutic treatment of inflammatory diseases of the mouth and/or the throat/pharynx, wherein the composition contains, in combination and in effective quantities in each case,
(a) marsh-mallow (*Althaea officinalis L.*) as a mucolytic drug
in a quantity ranging from 0.01% to 30% by weight based on the composition,
(b) polidocanol as a local anaesthetic
in a quantity ranging from 0.001% to 0.5% by weight based on the composition,
(c) polyhexanide as a surface disinfectant
in a quantity ranging from 0.001% to 0.5% by weight based on the composition,
wherein the composition contains the active ingredients and/or ingredients together with a pharmacologically and/or physiologically acceptable carrier and wherein the carrier is a combination based on water and propylene glycol and the weight-based ratio of water to propylene glycol ranges from 10:1 to 1:10,
wherein the mucolytic drug is used in the form of a liquid extract and/or a fluid extract with a drug to extract ratio ranging from 0.9:1 to 3:1, and
wherein the composition contains a preservative, wherein the preservative is potassium sorbate.

2. The composition for use according to claim 1, wherein the mucolytic drug is used in the form of an aqueous, alcoholic or aqueous-alcoholic extract.

3. The composition for use according to claim 1 or claim 2, wherein the mucolytic drug is used in the form of a liquid extract with a drug to extract ratio of approximately 1:1.

4. The composition for use according to any one of the preceding claims, wherein the composition contains the mucolytic drug in the form of its extract in a quantity ranging from 0.1 to 20% by weight based on the composition.

5. The composition for use according to any one of the preceding claims, wherein the surface disinfectant is polyhexanide hydrochloride.

6. The composition for use according to any one of the preceding claims, wherein the composition contains the preservative in a quantity ranging from 0.001% to 10% by weight, in particular ranging from 0.005% to 5% by weight, preferably ranging from 0.01% to 1% by weight, preferably ranging from 0.2% to 0.3% by weight, based on the composition.

7. The composition for use according to any one of the preceding claims, wherein the composition contains at least one further active ingredient, excipient, additive and/or ingredient.

8. The composition for use according to claim 7, wherein the at least one further active ingredient, excipient, additive and/or ingredient is selected from the group of processing aids, flavouring agents, flavours, sweeteners and sweetening agents, acidifying agents, stabilisers, vitamins, minerals and/or trace elements.

9. The composition for use according to any one of the preceding claims in the prophylactic and/or curative treatment of hoarseness and/or inflammatory diseases of the mouth and/or the throat/pharynx, in particular for use in the topical treatment of hoarseness or sore throats.

10. An application and/or administration device, in particular in the form of a spray applicator, wherein the application and/or administration device comprises a composition as defined in any one of the preceding claims.

## Revendications

1. Composition sous la forme d'un dosage liquide, destinée à être utilisée dans le traitement prophylactique ou thérapeutique de maladies inflammatoires de la cavité buccale et/ou de la cavité laryngopharyngée, dans laquelle la composition contient en association et en les quantités actives respectives
(a) de la guimauve (*Althaea officinalis L.*) comme agent mucolytique
en une quantité comprise dans la plage de 0,01 % à 30 % en poids, par rapport à la composition,
(b) du polidocanol comme anesthésique local
en une quantité comprise dans la plage de 0,001 % à 0,5 % en poids, par rapport à la composition,
(c) du polyhexanide comme désinfectant superficiel
en une quantité comprise dans la plage de 0,001 % à 0,5 % en poids, par rapport à la composition,
dans laquelle la composition contient les ingrédients actifs et/ou les ingrédients avec un support pharmacologiquement et/ou physiologiquement acceptable et dans laquelle le support est une combinaison à base d'eau et de propylèneglycol et le rapport pondéral de l'eau au propylèneglycol se situe dans la plage de 10:1 à 1:10,
dans laquelle l'agent mucolytique est mis en œuvre sous la forme d'un extrait liquide et/ou d'un extrait fluide avec un rapport agent/extrait compris dans la plage de 0,9:1 à 3:1 et
dans laquelle la composition contient un agent conservateur, l'agent conservateur étant du sorbate de potassium.

2. Composition à utiliser selon la revendication 1, dans laquelle l'agent mucolytique est mis en œuvre sous la forme d'un extrait aqueux, alcoolique ou hydroalcoolique.

3. Composition à utiliser selon la revendication 1 ou 2, dans laquelle l'agent mucolytique est mis en œuvre sous la forme d'un extrait liquide avec un rapport agent/extrait d'environ 1:1.

4. Composition à utiliser selon l'une quelconque des revendications précédentes, dans laquelle la composition contient l'agent mucolytique sous la forme de son extrait en une quantité dans la plage de 0,1 à 20 % en poids sur la base de la composition.

5. Composition à utiliser selon l'une quelconque des revendications précédentes, dans laquelle le désinfectant superficiel est le chlorhydrate de polyhexanide.

6. Composition à utiliser selon l'une quelconque des revendications précédentes, dans laquelle la composition contient l'agent conservateur en une quantité comprise dans la plage de 0,001 à 10 % en poids, en particulier dans la plage de 0,005 à 5 % en poids, préférentiellement dans la plage de 0,01 à 1 % en poids, de préférence dans la plage de 0,2 à 0,3 % en poids, par rapport à la composition.

7. Composition à utiliser selon l'une quelconque des revendications précédentes, dans laquelle la composition contient au moins un autre ingrédient actif, adjuvant, additif et/ou ingrédient.

8. Composition à utiliser selon la revendication 7, dans laquelle l'au moins un autre ingrédient actif, adjuvant, additif et/ou ingrédient est choisi dans le groupe constitué par les auxiliaires de mise en œuvre, les arômes, les agents de sapidité, les édulcorants et les produits édulcorants, les acidifiants, les stabilisants, les vitamines, les minéraux et/ou les oligo-éléments.

9. Composition à utiliser selon l'une quelconque des revendications précédentes dans le traitement prophylactique et/ou curatif de l'enrouement et/ou des maladies inflammatoires de la cavité buccale et/ou de la cavité laryngopharyngée, en particulier pour une utilisation dans le traitement topique de l'enrouement ou du mal de gorge.

10. Dispositif d'application et/ou d'administration, en particulier sous la forme d'un applicateur par pulvérisation, ledit dispositif d'application et/ou d'administration comprenant une composition, telle que définie dans l'une quelconque des revendications précédentes.
